# EUROPÄISCHE PATENTSCHRIFT

(11) **EP 3 549 552 B1**
(45) Veröffentlichungstag und Bekanntmachung des Hinweises auf die Patenterteilung: **23.09.2020**
(21) Anmeldenummer: 18166089.5
(22) Anmeldetag: 06.04.2018
(51) Int. Cl.: A61F 2/30, A61F 2/34

(54) **HÜFTGELENKSCHALE**
HIP JOINT CUP
CUPULE D'ARTICULATION DE LA HANCHE

(43) Veröffentlichungstag der Anmeldung: 09.10.2019
(73) Patentinhaber: Jossi Holding AG, 8546 Islikon (CH)
(72) Erfinder: Gugler, Christian, 8500 Frauenfeld (CH)
(74) Vertreter: Hepp Wenger Ryffel AG

(56) Entgegenhaltungen:
- EP-A1- 0 404 680
- EP-A1- 0 619 990
- WO-A1-2013/025308
- US-A- 5 876 456
- US-A1- 2013 310 945

## Beschreibung

Die Erfindung betrifft eine Hüftgelenkschale gemäss dem Oberbegriff von Anspruch 1. Derartige Implantate sind mit wenigstens einer zum Einsetzen einer Knochenschraube bestimmten Schraubenöffnung versehen, welche einen sich verjüngenden Querschnitt und wenigstens ein Innengewinde aufweist, in welches zum Verschliessen der Schraubenöffnung eine Verschlussschraube einschraubbar ist.

Hüftgelenkschalen werden häufig als zementfreie Einpressschalen ausgebildet, deren Schalenaussenseite derart ausgebildet ist, dass eine biologische Fixierung durch Einwachsen von Knochengewebe und damit eine langzeitstabile Verankerung des Implantats ermöglicht wird. Die Primärstabilität einer Schale kann durch zusätzliche Knochenschrauben erhöht werden. Die nicht verwendeten Schraubenöffnungen sollten jedoch durch entsprechende Verschlussschrauben verschlossen sein, um einen Austausch von Partikeln und Flüssigkeit zwischen Gewinde und Innenraum der Hüftgelenkschale zu verhindern. Ausserdem ist es je nach den anatomischen Gegebenheiten erforderlich, dass die Knochenschraube in einem Winkel von ca. +/- 20° zur Längsmittelachse der Schraubenöffnung in den Knochen eingeschraubt werden kann. Diese Voraussetzungen erfordern ersichtlicherweise einen dichten Verschluss der Schraubenöffnung, eine leichte Montage und Demontage der Verschlussschraube und eine optimale Ausrichtung der Knochenschraube.

Durch die US 2013/0310945 A1 ist eine Hüftgelenkschale mit Schraubenöffnungen und korrespondierenden Verschlussschrauben bekannt geworden, wobei ein zylindrisches Schraubgewinde entweder nahe an der Innenseite der Schale oder nahe an der Aussenseite der Schale angeordnet sein kann. Die Schraubenöffnung verfügt jeweils wahlweise auch über einen sphärischen Abschnitt, der sich gegen die Aussenseite der Schale hin verjüngt.

Durch die CN 206080772 U ist eine Hüftgelenkschale bekannt geworden, welche ebenfalls über sich sphärisch verjüngende Schraubenöffnungen verfügt. Ein Innengewinde ist unmittelbar im Bereich der Innenseite der Schale angeordnet. Eine Fixierschraube wird bei eingesetzter Knochenschraube eingeschraubt, wobei ein halbkugelförmiger Ansatz Druck auf den Schraubenkopf ausübt und dadurch die Knochenschraube fixiert.

Bekannte Hüftgelenkschalen weisen bezogen auf die Längsmittelachse der Schraubenöffnung eine serielle Anordnung der sphärischen Auflage für den Schraubenkopf und des Innengewindes für die Verschlussschraube auf. Dadurch entsteht das Problem, dass die gesamte Wanddicke der Schale erhöht werden muss, was aus Gründen des damit verbundenen erhöhten Knochenverlustes nicht gewünscht ist. Bedingt durch eine allfällige Schrägstellung der Knochenschraube ist ausserdem die vollständige Auflage auf dem sphärischen Abschnitt und damit auch die Dichtigkeit nicht mehr gegeben. Dabei ist ausserdem noch zu berücksichtigen, dass die Länge des Innengewindes wenigstens mehr als eine Umdrehung der Verschlussschraube erlauben sollte, um einen sicheren Halt und eine zuverlässige Abdichtung zu gewährleisten. Bedingt durch das Gewinde im Schraubenloch kann bereits eine geringe Schrägstellung der Knochenschraube zu einer unvollständigen Auflage des Schraubenkopfs im Schraubentrichter führen. Dies kann zu mechanischer Instabilität sowie zu Undichtigkeit führen.

Es ist daher eine Aufgabe der Erfindung, eine Hüftgelenkschale der eingangs genannten Art zu schaffen, welche einfach herzustellen ist und bei welcher die Gesamtdicke der Schale inklusive einer strukturierten oder beschichteten Aussenseite so klein wie möglich gehalten werden kann, ohne dass dabei ein sicherer Halt der Verschlussschraube und eine zuverlässige Abdichtung beeinträchtigt werden. Der zylindrische Anteil der Schraubenöffnung für die Knochenschraube soll dabei minimiert oder ganz eliminiert werden, ohne dass die Auflagefläche des Kopfes der Knochenschraube oder die Dichtwirkung beeinträchtigt werden. Schliesslich soll innerhalb der Schraubenöffnung auch nach wie vor ein ausreichender Bewegungsspielraum für den Schraubenkopf bei unterschiedlichen Winkelstellungen der Knochenschraube verbleiben.

Diese Aufgaben werden durch eine Hüftgelenkschale gelöst, welche die Merkmale im Anspruch 1 aufweist. Die Hüftgelenkschale hat dabei eine Innenseite und eine Aussenseite und wenigstens eine zum Einsetzen einer Knochenschraube bestimmte Schraubenöffnung. Diese weist wenigstens einen, den Querschnitt gegen die Aussenseite verjüngenden sphärischen Abschnitt auf, der als Widerlager für den Kopf der Knochenschraube dient. Ausserdem weist die Schraubenöffnung ein Innengewinde zum Verschliessen der Schraubenöffnung mit einer Verschlussschraube auf. Dieses Innengewinde ist dabei im sphärischen Abschnitt angeordnet, insbesondere ausschliesslich im sphärischen Abschnitt.

Durch die erfindungsgemässe Anordnung des Innengewindes wird die Aufteilung der Schalentiefe in einen sphärischen Abschnitt und in einen Gewindeabschnitt aufgehoben und der sphärische Abschnitt übernimmt zumindest teilweise auch noch die Gewindefunktion, ohne dass dadurch die Funktion als dichtes Auflager oder Widerlager für den Kopf der Knochenschraube aufgehoben wird. Ersichtlicherweise erlaubt dies eine Minimierung der Schalendicke, ohne dass dadurch die Funktion des Schraubentrichters beeinträchtigt wird.

Das Innengewinde kann dabei ein sphärisches oder ein konisches Gewinde sein. Diese Gewindetypen sind im Maschinenbau oder in der Feinmechanik eher ungewöhnlich, da sie besondere Anforderungen an den Herstellungsprozess stellen. Es handelt sich dabei um Gewinde, bei denen die Gewindespirale nicht in einer zylindrischen, sondern in einer sphärischen oder kegelförmigen Oberfläche angeordnet ist. Unter dem Begriff einer sphärischen Oberfläche sind dabei auch solche Oberflächen zu verstehen, die nicht exakt kugelförmig, sondern beispielsweise auch ellipsoid ausgebildet sind. Alternativ kann das Innengewinde aber auch ein zylindrisches Gewinde sein. Der Bereich des Innengewindes behindert die eingesetzte Knochenschraube nicht und dieser Bereich steht auch für das Anliegen des Schraubenkopfs der Knochenschraube zur Verfügung. Auch bei dünnwandigen Schalen kann somit ein Innengewinde mit einer ausreichenden Länge angeordnet werden. Der sphärische Abschnitt ermöglicht ein Ausschwenken der Knochenschraube aus der Längsmittelachse der Schraubenöffnung in alle Richtungen, wobei der sphärische Schraubenkopf stets auf einer bestimmten Umfangsfläche am sphärischen Abschnitt der Schraubenöffnung anliegt, ohne dass dabei das Innengewinde diese Bewegung behindert.

Es ist ausserdem besonders vorteilhaft, wenn das Innengewinde derart ausgebildet ist, dass bezogen auf den Querschnitt zwischen einzelnen Gewindegängen sphärische Teilabschnitte verbleiben. Auf diese Weise wird eine möglichst grosse Auflagefläche und Dichtfläche im sphärischen Abschnitt erzielt und es wird verhindert, dass der Kopf der Knochenschraube im Gewindebereich ausschliesslich auf den Spitzen des Innengewindes aufliegt.

Es kann vorteilhaft sein, wenn sich an den sphärischen Abschnitt auf der Seite mit dem kleineren Innendurchmesser gegen die Aussenseite hin ein zylindrischer Abschnitt anschliesst, dessen Länge minimal ist und vorzugsweise 0.1 mm bis 0.4 mm beträgt. Über diesen zylindrischen Abschnitt wird verhindert, dass sich das Innengewinde bis zu einer Aussenfläche des Implantats erstreckt, womit unerwünschte scharfe und brüchige Kanten entstehen könnten. Ausserdem kann die Verschlussschraube einen korrespondierenden zylindrischen Abschnitt aufweisen, der passgenau in den zylindrischen Abschnitt der Schraubenöffnung eingefügt werden kann. Die gesamte axiale Länge der Schraubenöffnung kann beispielsweise im Bereich zwischen 1,5 bis 5 mm liegen. Der Innendurchmesser des zylindrischen Abschnitts kann dabei entsprechend den gängigen Knochenschrauben im Bereich von 4 bis 7 mm liegen.

Es ist weiter besonders vorteilhaft, wenn sich das Innengewinde im sphärischen Abschnitt nicht bis zum zylindrischen Abschnitt erstreckt. Damit verbleibt zwischen dem Ende des Gewindegangs und dem Anfang des zylindrischen Abschnitts in jedem Fall ein sphärischer Abschnitt, der sich über den gesamten Umfang von 360° erstreckt. Dieser Abschnitt ist entscheidend für die angestrebte Dichtwirkung bei eingeschraubter Verschlussschraube bzw. bei eingesetzter Knochenschraube. Die Distanz zwischen dem äussersten Gewindegang und dem Beginn des zylindrischen Abschnitts sollte wenigstens 0,1 mm oder mehr betragen.

Weitere Vorteile können erzielt werden, wenn sich der sphärische Abschnitt bis zur Innenseite der Hüftgelenkschale erstreckt. Auf diese Weise steht fast die gesamte Schalendicke als Widerlagerfläche und als Bewegungsraum für den Schraubenkopf der Knochenschraube zur Verfügung. Selbstverständlich wäre es jedoch denkbar, dass der sphärische Abschnitt auch gegen die Innenseite hin in einen zylindrischen oder in einen konischen Abschnitt übergeht.

Vorteilhaft ist das Innengewinde ausserdem derart angeordnet, dass bezogen auf die Einschraubrichtung der Anfang des Innengewindes im Abstand zum grössten Durchmesser des sphärischen Abschnitts angeordnet ist. Damit ist gewährleistet, dass die Schraubenöffnung stets einen ausreichend grossen Abschnitt ohne Innengewinde aufweist. Dieser Abschnitt liegt im Bereich des grösseren Innendurchmessers und damit in einem Bereich des grössten Ausserdurchmessers am Kopf der Knochenschraube. Ausserdem wird dadurch gewährleistet, dass bei eingeschraubter Verschlussschraube kein Innengewindegang sichtbar ist, auch wenn die Dicke der Verschlussschraube kleiner ist als die maximale Länge der Schraubenöffnung.

Das Innengewinde kann ein eingängiges Gewinde mit wenigstens einem vollständigen Gewindeumgang sein. In bestimmten Fällen und insbesondere bei dünnwandigen Implantaten kann es aber auch zweckmässig sein, wenn das Innengewinde ein mehrgängiges Gewinde, insbesondere ein zweigängiges oder ein dreigängiges Gewinde ist. Die einzelnen Gewindegänge können sich dabei auch nur über einen Bruchteil eines vollständigen Gewindeumgangs erstrecken, ohne dass die Stabilität der Gewindeverbindung darunter leidet. Ausserdem ist das Innengewinde vorteilhaft ein Spitzgewinde, das für sphärische oder konische Gewinde geeignet ist. Auch andere Gewindeformen wie z.B. Rundgewinde, Trapezgewinde oder Sägezahngewinde wären jedoch denkbar.

Vorzugsweise ist die Winkelhalbierende des Flankenwinkels an einem Spitzgewinde bezogen auf die Längsmittelachse der Schraubenöffnung von der Eintrittsseite gegen die Austrittsseite geneigt. Die Gewindeflanken am Gewinde verlaufen somit nicht im rechten Winkel zur Längsmittelachse, sondern sie sind geneigt. Damit kann trotz der Krümmung des sphärischen Abschnitts ein möglichst gleichförmiger Gewindegang über die gesamte Gewindelänge erzielt werden.

Die Hüftgelenkschale kann mit wenigstens einer Verschlussschraube bestückt sein, welche ein Aussengewinde aufweist, das in das Innengewinde der Schraubenöffnung einschraubbar ist und das wenigstens einen zum sphärischen Abschnitt der Schraubenöffnung korrespondierenden sphärischen Aussenabschnitt aufweist. Besonders vorteilhaft ist dabei auch das Aussengewinde der Verschlussschraube vorzugsweise ausschliesslich im sphärischen Aussenabschnitt der Verschlussschraube angeordnet. Damit werden im Hinblick auf die Verschlussschraube die gleichen Vorteile realisiert wie bei der Schraubenöffnung der Schale, nämlich ein Zusammenfallen von sphärischem Bereich und Gewindebereich und damit eine flache Ausgestaltung der Verschlussschraube.

Die Verschlussschraube kann dabei als Scheibe ausgebildet sein, deren Höhe kleiner ist als die gesamte axiale Länge der Schraubenöffnung. Dadurch ist gewährleistet, dass die in die Schraubenöffnung eingeschraubte Verschlussschraube die eingesetzte innere Gelenkschale nicht behindert. Die Ausbildung als flache Scheibe ist ausserdem herstellungstechnisch einfacher als die Ausbildung als gekrümmter Schalenabschnitt.

Weitere Vorteile an der Verschlussschraube können erzielt werden, wenn sie einen sphärischen Abschnitt aufweist, der bezogen auf die Einschraubrichtung vor dem Aussengewinde angeordnet ist und wenn dieser Abschnitt bei eingeschraubter Verschlussschraube mit einem sphärischen Abschnitt der Schraubenöffnung korrespondiert. Auf diese Weise wird im Bereich des geringeren Durchmessers der sich verjüngenden Schraubenöffnung bei eingeschraubter Verschlussschraube eine Dichtfläche erzielt, welche bei entsprechender Kongruenz der sphärischen Abschnitte flüssigkeitsdicht ist. Durch das Anziehen der Verschlussschraube wird ausserdem ein Anpressdruck auf diese Dichtfläche ausgeübt. In einer vorteilhaften Ausgestaltung kann dieser sphärische Abschnitt beispielsweise einen Radius zwischen 3 bis 5 mm aufweisen. Diese Radiusangabe gilt selbstverständlich auch für den Radius des sphärischen Abschnitts an der Schraubenöffnung. Die scheibenförmige Verschlussschraube kann beispielsweise eine Dicke bzw. Höhe im Bereich zwischen 2 bis 5 mm aufweisen. Das Aussengewinde der Verschlussschraube kann analog zum Innengewinde der Schraubenöffnung ebenfalls ein Spitzgewinde sein wobei die Flanke bzw. die Winkelhalbierende des Flankenwinkels auf die gleiche Weise geneigt ist wie am Innengewinde.

Die Verschlussschraube kann ein in eine Oberfläche eingelassenes Mitnahmeprofil, insbesondere einen Innensechskant oder einen Innenvierkant zum Ansetzen eines Einschraubwerkzeuges aufweisen. Selbstverständlich wären auch alternative Mitnahmeprofile denkbar wie z.B. voneinander beabstandete Bohrungen zum Einsetzen eines Lochschlüssels, vertiefte oder erhabene Verzahnungen usw. Analog zum Innengewinde der Schraubenöffnung kann auch das Aussengewinde der Verschlussschraube ein eingängiges Gewinde mit wenigstens einem vollständigen Gewindegang oder ein mehrgängiges Aussengewinde, insbesondere ein zweigängiges oder ein dreigängiges Gewinde sein.

Die Hüftgelenkschale und/oder die Verschlussschraube können aus einem Polymer, insbesondere aus einem mit Titan beschichteten Polymer gefertigt sein. Insbesondere die Verschlussschraube könnte dabei kostengünstig als Spritzgussteil hergestellt werden. Die Beschichtung der Bauteile beispielsweise mit Titan könnte optional nur auf derjenigen Seite erfolgen, welche mit Knochenmaterial in Berührung kommt. Geeignete biokompatible polymere Werkstoffe und entsprechende Beschichtungsverfahren sind dem Fachmann allgemein bekannt.

Die erfindungsgemässe Hüftgelenkschale ist vorzugsweise an jeder Schraubenöffnung bereits mit einer Verschlussschraube wie oben beschrieben bestückt. Vorzugsweise gehört zu dieser Hüftgelenkschale auch eine entsprechende Anzahl passender Knochenschrauben, wobei die Schraubenöffnung der Hüftgelenkeschale und der Schraubenkopf der Knochenschraube derart ausgebildet sind, dass die Knochenschraube bezogen auf die Längsmittelachse der Schraubenöffnungen in verschiedenen Winkelstellungen einschraubbar ist. Bei Hüftgelenkschalen handelt es sich dabei vorteilhaft um Spongiosaschrauben mit einem Flach- oder Linsenkopf und einem gegen das Gewinde gerichteten sphärischen Abschnitt.

Der sphärische Abschnitt der Schraubenöffnung in der Hüftgelenkschale, der sphärische Aussenabschnitt der Verschlussschraube und der sphärische Schraubenkopfabschnitt der Knochenschraube sind derart aufeinander abstimmt, dass die Schraubenöffnung sowohl bei eingeschraubter Verschlussschraube, als auch bei eingesetzter Knochenschraube fluiddicht verschliessbar ist. Dabei spielt es im Hinblick auf die Dichtigkeit keine Rolle, ob die vorhandenen Schraubenöffnungen mit Verschlussschrauben oder mit Knochenschrauben bestückt sind.

Eine derartige Hüftgelenkschale kann zwischen dem äquatorialen Bereich und dem Polbereich in Umfangsrichtung mehrere Schraubenöffnungen aufweisen, vorzugsweise 3 bis 5 Schraubenöffnungen, die aber nicht notwendigerweise auf dem gleichen Schalendurchmesser angeordnet sein müssen. Im Polbereich können derartige Schalen ausserdem über eine weitere Öffnung verfügen, welche dem Ansetzen eines Manipulationswerkzeugs zum Einsetzen der Schale in den Knochen dient.

Die vorstehend beschriebenen Hüftgelenkschalen weisen auf der Oberfläche vorteilhaft eine Strukturierung auf, welche eine feste Verbindung der Hüftgelenkschale mit dem Knochenmaterial begünstigt. Dabei kann es sich um eine Makrostruktur, um eine poröse Beschichtung oder um eine Kombination der beiden Varianten handeln. Die Materialstärke der Strukturierung und/oder der Beschichtung erhöht zwar die Gesamtdicke der Hüftgelenkschale, sie trägt jedoch nicht dazu bei, die Funktion der Verschlussschraube oder der Knochenschraube zu begünstigen.

Weitere Einzelheiten der Schalenkonstruktion sind dem Fachmann bekannt und geläufig. Hüftgelenkschale und Verschlussschrauben können aus den üblichen Werkstoffen nach ISO 5832, insbesondere aus Titan oder Titanlegierungen gefertigt sein. In Ausnahmefällen könnten auch keramische oder polymere Werkstoffe zum Einsatz kommen. Auch eine Paarung unterschiedlicher Werkstoffe für das Implantat und die Verschlussschraube wäre denkbar. Weitere Einzelmerkmale und Vorteile der Erfindung ergeben sich aus der Beschreibung des nachfolgenden Ausführungsbeispiels und aus den Zeichnungen. Es zeigen:
- Figur 1: einen Querschnitt durch eine Hüftgelenkschale ohne eingesetzte Verschlussschraube,
- Figur 2: eine Draufsicht auf die Hüftgelenkschale gemäss Figur 1,
- Figur 3: das Detail X gemäss Figur 1,
- Figur 4: einen Querschnitt durch eine Verschlussschraube in vergrösserter Darstellung,
- Figur 5: das Detail Y gemäss Figur 4,
- Figur 6: eine Draufsicht auf die Verschlussschraube gemäss Figur 4,
- Figur 7: eine perspektivische Darstellung der Verschlussschraube gemäss Figur 4,
- Figur 8: einen Querschnitt durch eine in eine Schraubenöffnung eingeschraubte Verschlussschraube,
- Figur 9: die Ansicht einer Knochenschraube,
- Figur 10: eine vergrösserte Darstellung einer in eine Schraubenöffnung eingesetzten Knochenschraube,
- Figur 11: einen Querschnitt durch die Hüftgelenkschale gemäss Figur 1 mit eingeschraubter Verschlussschraube,
- Figur 12: einen Querschnitt durch die Hüftgelenkschale gemäss Figur 1 mit eingesetzter Knochenschraube, und
- Figur 13: einen stark vereinfachten Teilquerschnitt durch eine Schraubenöffnung mit einem zylindrischen Innengewinde.

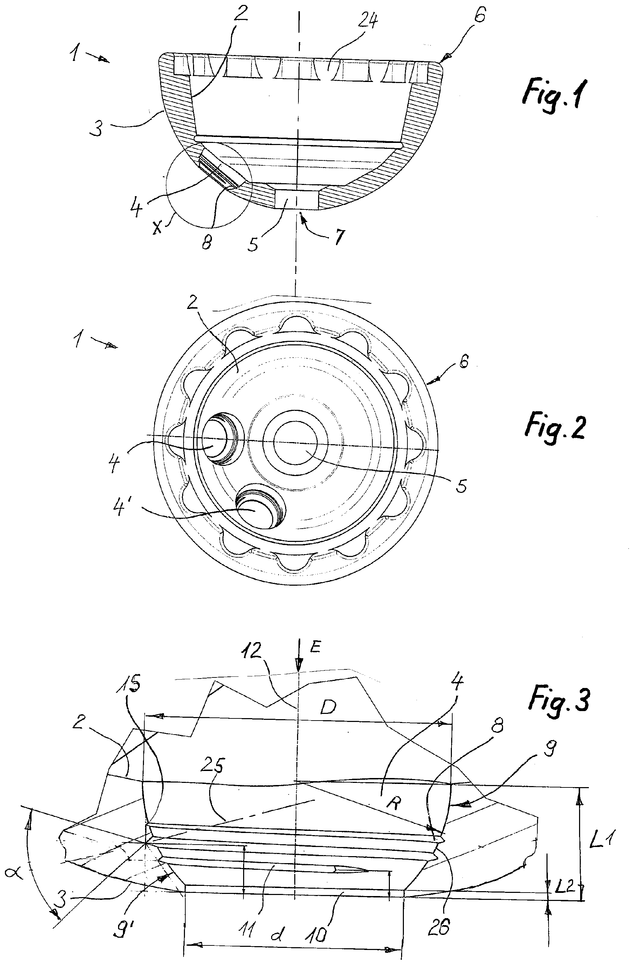

Wie in den Figuren 1 und 2 dargestellt, besteht eine Hüftgelenkschale 1 aus einer etwa halbkugelförmigen Schale mit einer Innenseite 2 und mit einer Aussenseite 3. Die Hüftgelenkschale wird auch als Pfanne bezeichnet, die einen Äquatorialbereich 6 und einen Polbereich 7 aufweist. Im Polbereich 7 ist eine Werkzeugöffnung 5 vorgesehen, an der ein Manipulationswerkzeug angesetzt werden kann. Auch die Vertiefungen 24 im Äquatorialbereich dienen der Manipulation der Schale bzw. der Befestigung der hier nicht dargestellten Innenschale, in welcher letztlich die Gelenkkugel gelagert ist. In der Regel ist die Aussenseite 3 der Hüftgelenkschale nicht exakt halbkugelförmig ausgebildet, sondern im Querschnitt leicht elliptisch, so dass sich im Polbereich 7 eine leichte Abflachung ergibt.

In der Hüftgelenkschale sind über den Schalenumfang verteilt mehrere Schraubenöffnungen 4 angeordnet, von denen der Einfachheit halber lediglich zwei (4, 4') eingezeichnet sind. Die Konfiguration dieser Schraubenöffnungen ist in Figur 3 genauer dargestellt. Im Querschnitt verjüngt sich die Schraubenöffnung von der Innenseite 2 nach der Aussenseite 3 und zwar durch einen sphärischen Abschnitt 9, der sich vom grössten Durchmesser D auf der Innenseite 2 bis zum kleinsten Durchmesser d kurz vor der Aussenseite 3 erstreckt. Der sphärische Abschnitt 9 hat einen Radius R, der dem Kopfradius einer nachstehend noch beschriebenen Knochenschraube angepasst ist. Kurz vor der Aussenseite 3 weist die Schraubenöffnung 4 noch einen kurzen zylindrischen Abschnitt 10 auf, der im Vergleich zur Gesamtlänge L1 der Schraubenöffnung nur eine relativ geringe Länge L2 aufweist.

Im sphärischen Abschnitt 9 ist ein Innengewinde 11 angeordnet, welches der Krümmung des sphärischen Abschnitts folgt. Im dargestellten Ausführungsbeispiel handelt es sich um ein eingängiges Innengewinde mit wenigstens zwei vollständigen Gewindeumgängen. Wie aus dem Querschnitt ersichtlich ist, handelt es sich dabei um ein Spitzgewinde. Der Flankenwinkel α eines Gewindegangs hat eine Winkelhalbierende 25, welche relativ zur Längsmittelachse 12 der Schraubenöffnung 4 geneigt ist und zwar gegen die Aussenseite der Schale, sodass der Querschnitt der Innengewindeflanke der Krümmung des sphärischen Abschnitts 9 folgt. Ersichtlicherweise erstreckt sich das Innengewinde 8 nicht über die gesamte Länge L1 der Schraubenöffnung 4. Anfang und Ende des Gewindegangs 11 sind so angeordnet, dass sie jeweils in einem Abstand zur Innenseite 2 und zur Aussenseite 3 der Hüftgelenkschale angeordnet sind. Insbesondere ist der Anfang 15 des Innengewindes bezogen auf die Einschraubrichtung E im Abstand zum grössten Durchmesser D und damit zur Innenseite 2 der Hüftgelenkschale angeordnet. Dabei verbleibt insbesondere im Bereich des grössten Durchmessers D ein sphärischer Abschnitt 9 ohne Innengewinde, der etwa 40% bis 50% der gesamten Länge L1 entspricht. Im Bereich des kleineren Durchmessers d verbleibt ebenfalls ein sphärischer Abschnitt 9' ohne Innengewinde, dessen Länge wenigstens 0.1 mm oder mehr betragen muss.

Das Innengewinde 8 bzw. die einzelnen Gewindegänge 11 sind derart in den sphärischen Abschnitt 9 eingearbeitet, dass bezogen auf die Längsmittelachse 12 im Querschnitt sphärische Teilabschnitte 26 verbleiben. Auf diese Weise ist gewährleistet, dass praktisch über und unter jedem einzelnen Innengewindegang ein sphärischer Abschnitt oder Teilabschnitt angeordnet ist, der eine Tragfunktion ausüben kann.

Unabhängig von der in Figur 3 dargestellten Detailkonfiguration der Schraubenöffnung kann die Steigung für ein eingängiges Gewinde zwischen 0,3mm bis 0,9mm, vorteilhaft zwischen 0,4mm und 0,5mm betragen. Bei mehrgängigen Gewinden beträgt die Gewindesteigung ein entsprechendes Vielfaches von diesen Werten. Ausserdem ist es vorteilhaft, wenn das Innengewinde unabhängig vom Gewindetyp nur soweit in den sphärischen Abschnitt eindringt, dass die ursprüngliche sphärische Fläche zu 50% bis 70% erhalten bleibt. Schliesslich wäre es denkbar, dass insbesondere im Falle eines Spitzgewindes das Innengewinde auch durch eine selbstschneidende Verschlussschraube erzeugt werden kann.

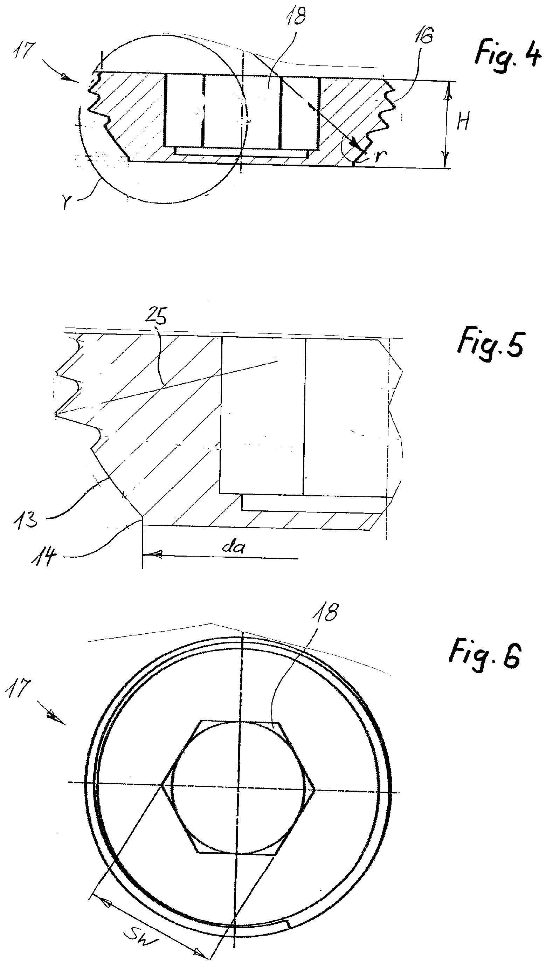

Die Figuren 4 bis 7 zeigen eine Verschlussschraube 17, mit welcher eine Schraubenöffnung 4 oder 4' in der Hüftgelenkschale 1 verschlossen werden kann. Die Verschlussschraube hat eine Gesamthöhe H, welche in jedem Fall kleiner ist als die Gesamtlänge L1 einer Schraubenöffnung. Die Verschlussschraube verfügt ebenfalls über einen sphärischen Abschnitt 13, dessen Radius r dem Radius R des sphärischen Abschnitts 9 in der Schraubenöffnung entspricht. Ausserdem verfügt die Verschlussschraube über einen zylindrischen Abschnitt 14, dessen Aussendurchmesser da mit geringem Untermass dem Innendurchmesser d an der Schraubenöffnung 4 entspricht. Das Aussengewinde 16 der Verschlussschraube ist korrespondierend zum sphärischen Innengewinde 8 in der Schraubenöffnung ausgebildet. Ersichtlicherweise bedeutet dies, dass die Winkelhalbierende 25 eines Flankenwinkels auf die gleiche Weise geneigt ist wie am Innengewinde.

Zum Eindrehen und Anziehen der Verschlussschraube 17 ist ein Innensechskant 18 angeordnet, der sich fast über die gesamte Höhe H der Verschlussschraube erstreckt. Der Innensechskant hat eine Schlüsselweite SW, dessen Dimension den gängigen genormten Schraubwerkzeugen entspricht.

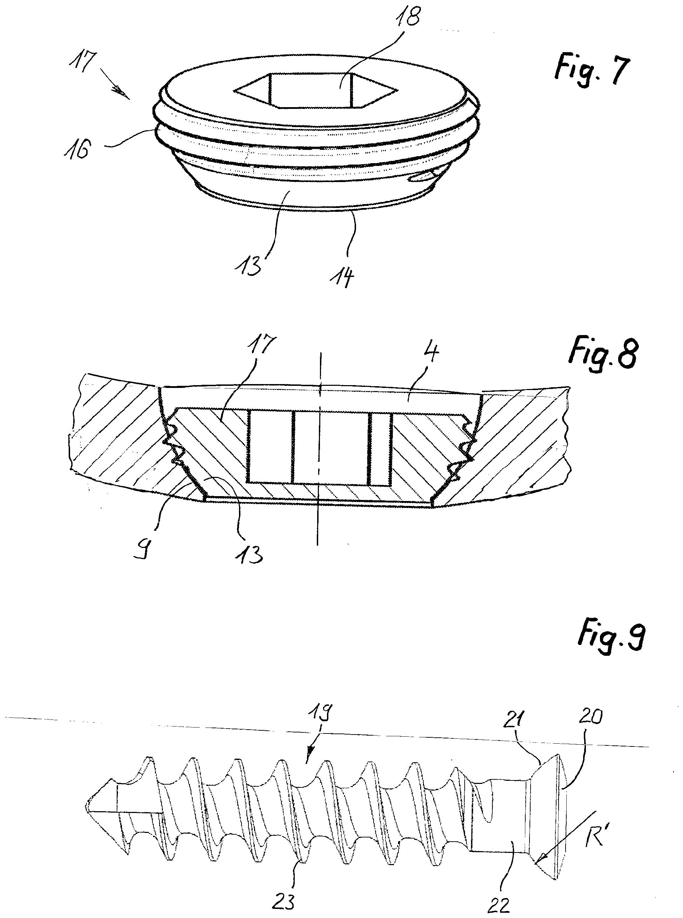

Figur 8 zeigt im Querschnitt eine Verschlussschraube 17, welche in eine Schraubenöffnung 4 eingeschraubt ist. Wie dargestellt liegen dabei die sphärischen Abschnitte 9 der Schraubenöffnung 4 und 13 der Verschlussschraube 17 formschlüssig und dichtend aufeinander, sofern diese Abschnitte nicht durch Schraubengänge unterbrochen sind. Es stehen ausserdem genügend Gewindegänge zur Verfügung, um eine sichere und feste Verbindung zu gewährleisten. Trotzdem steht nach dem Entfernen einer Verschlussschraube 17 der gesamte sphärische Abschnitt 9 als Widerlager für die Knochenschraube zur Verfügung.

Eine typische Knochenschraube 19 in der Form einer Spongiosaschraube ist in Figur 9 stark vergrössert dargestellt. Sie verfügt über einen linsenförmigen Schraubenkopf 20, an den sich ein zylindrischer Schraubenhals 22 anschliesst. Ein Gewindegang 23 erstreckt sich fast bis zum Ende der Schraube. Am Schraubenkopf 20 und dem Gewindegang 23 zugeneigt ist ein sphärischer Abschnitt 21 angeordnet. Dessen Radius R' entspricht etwa dem Radius R des sphärischen Abschnitts 9 in der Schraubenöffnung 4.

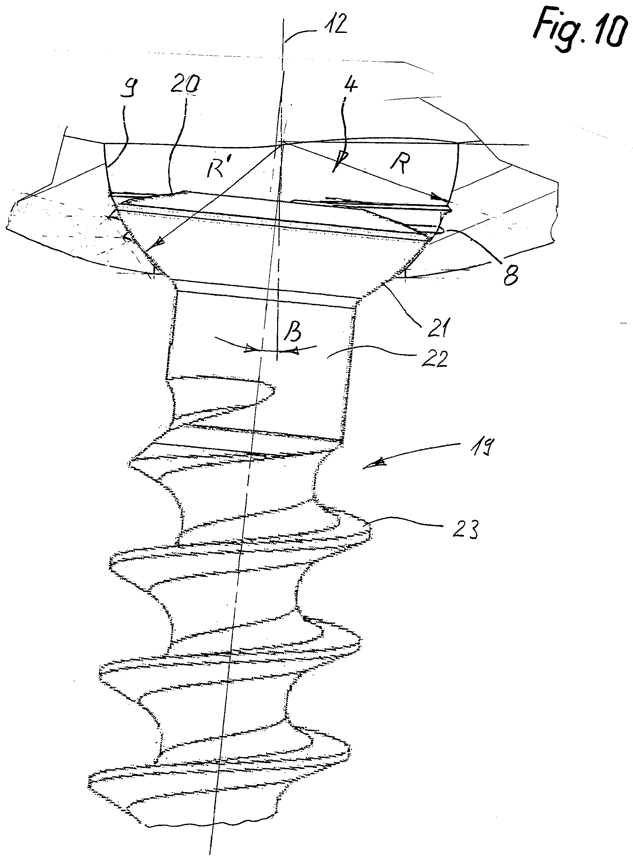

Wie insbesondere aus Figur 10 ersichtlich ist, erlaubt dies ein Einsetzen der Knochenschraube 19 in die Schraubenöffnung 4 derart, dass sie je nach den anatomischen Gegebenheiten innerhalb eines bestimmten Spektrums in einem Neigewinkel β relativ zur Längsmittelachse 12 der Schraubenöffnung in den Knochen eingeschraubt werden kann. Trotz des vorhandenen Innengewindes 8 verbleibt eine ausreichende Anlagefläche zwischen den sphärischen Abschnitten 9 der Schraubenöffnung 4 und 21 der Knochenschraube 19, um auch einen ausreichenden Kraftschluss zwischen Schraube und Hüftgelenkschale zu gewährleisten.

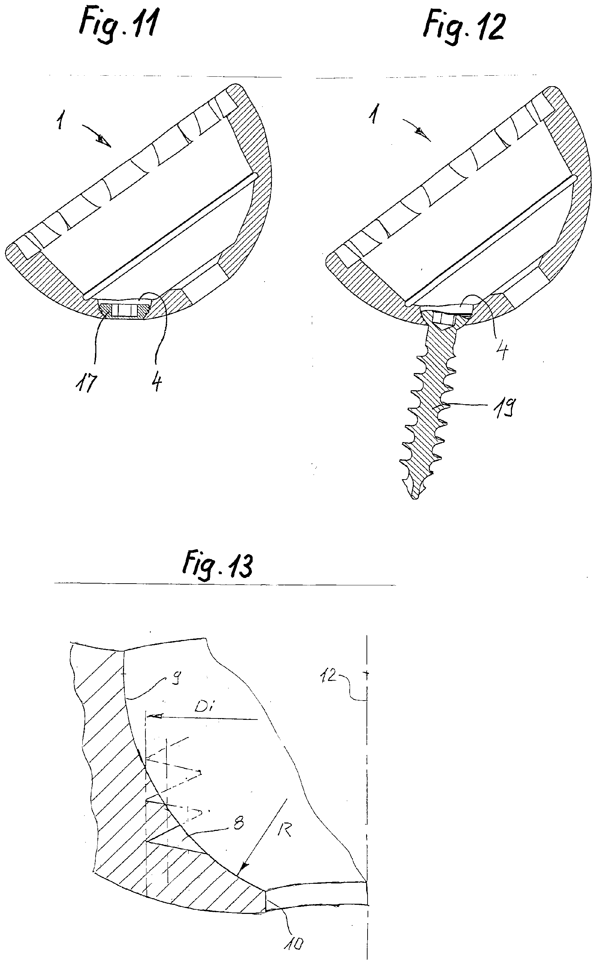

Die Figuren 11 und 12 zeigen nochmals gegenübergestellt die Situation einer mit einer Verschlussschraube 17 verschlossenen Schraubenöffnung 4 und einer in die Schraubenöffnung 4 eingesetzten Knochenschraube 19. Selbstverständlich sind die Dimensionen jeweils variabel und anstelle einer Spongiosaschraube könnten auch andere Schraubentypen verwendet werden. Werkseitig können die Verschlussschrauben 17 der Hüftgelenkschale beigelegt werden oder sie können unter sterilen Bedingungen bereits montiert werden.

In Figur 13 ist symbolisch die extreme Situation dargestellt, bei welcher ein zylindrisches Innengewinde 8 in den sphärischen Abschnitt 9 eingearbeitet ist. Dieses Innengewinde verfügt über einen Fussdurchmesser Di, dessen Hüllkurve den sphärischen Abschnitt 9 unter einem flachen Winkel schneidet. Ersichtlicherweise muss bei dieser Konstellation die Gesamtlänge des Innengewindes grösser sein als beispielsweise bei einem sphärischen Innengewinde, um noch eine ausreichende Anzahl Gewindegänge zu erzielen.

## Patentansprüche

1. Hüftgelenkschale (1) mit einer Innenseite (2) und einer Aussenseite (3) und mit wenigstens einer zum Einsetzen einer Knochenschraube (19) bestimmten Schraubenöffnung (4), welche wenigstens einen, den Querschnitt gegen die Aussenseite verjüngenden sphärischen Abschnitt als Widerlager für die Knochenschraube und wenigstens ein Innengewinde (8) zum Verschliessen der Schraubenöffnung mit einer Verschlussschraube (17) aufweist, **dadurch gekennzeichnet, dass** das Innengewinde (8) im sphärischen Abschnitt (9) angeordnet ist.

2. Hüftgelenkschale nach Anspruch 1, wobei das Innengewinde (8) ein sphärisches oder ein konisches oder ein zylindrisches Gewinde ist.

3. Hüftgelenkschale nach Anspruch 1 oder 2, wobei das Innengewinde (8) derart ausgebildet ist, dass bezogen auf den Querschnitt zwischen einzelnen Gewindegängen (11) sphärische Teilabschnitte (26) verbleiben.

4. Hüftgelenkschale nach einem der Ansprüche 1 bis 3, wobei sich an den sphärischen Abschnitt (9) gegen die Aussenseite (3) hin ein zylindrischer Abschnitt (10) anschliesst, dessen Länge (1), vorzugsweise 0.1 mm bis 0.4 mm beträgt.

5. Hüftgelenkschale nach Anspruch 4, wobei sich das Innengewinde (8) nicht bis zum zylindrischen Abschnitt (10) erstreckt.

6. Hüftgelenkschale nach einem der Ansprüche 1 bis 5, wobei sich der sphärische Abschnitt (9) bis zur Innenseite (2) erstreckt.

7. Hüftgelenkschale nach einem der Ansprüche 1 bis 6, wobei das Innengewinde derart angeordnet ist, dass bezogen auf die Einschraubrichtung der Anfang (15) des Innengewindes im Abstand zum grössten Durchmesser (D) des sphärischen Abschnitts (9) angeordnet ist.

8. Hüftgelenkschale nach einem der Ansprüche 1 bis 7, wobei das Innengewinde (8) ein eingängiges Gewinde mit wenigstens einem vollständigen Gewindeumgang ist.

9. Hüftgelenkschale nach einem der Ansprüche 1 bis 7, wobei das Innengewinde (8) ein mehrgängiges Gewinde, insbesondere ein zweigängiges oder ein dreigängiges Gewinde ist.

10. Hüftgelenkschale nach einem der Ansprüche 1 bis 9, wobei das Innengewinde (8) ein Spitzgewinde ist.

11. Hüftgelenkschale nach Anspruch 10, wobei die Winkelhalbierende (25) des Flankenwinkels (α) bezogen auf die Längsmittelachse (12) der Schraubenöffnung (4) von der Eintrittsseite gegen die Austrittsseite geneigt ist.

12. Hüftgelenkschale nach einem der Ansprüche 1 bis 11, umfassend wenigstens eine Verschlussschraube (17) zum Verschliessen der wenigstens einen Schraubenöffnung (4), wobei die Verschlussschraube ein Aussengewinde (16) aufweist, das in das Innengewinde (8) einschraubbar ist, sowie wenigstens einen zum sphärischen Abschnitt (9)der Schraubenöffnung (4) korrespondierenden sphärischen Aussenabschnitt (13).

13. Hüftgelenkschale nach Anspruch 12, wobei das Aussengewinde (16) der Verschlussschraube (17) im sphärischen Aussenabschnitt (13) angeordnet ist.

14. Hüftgelenkschale nach Anspruch 12 oder 13, wobei die Verschlussschraube (17) als Scheibe ausgebildet ist, deren Höhe (H) kleiner ist als die gesamte axiale Länge (L) der Schraubenöffnung (4).

15. Hüftgelenkschale nach einem der Ansprüche 12 bis 14, wobei die Verschlussschraube (17) ein in eine Oberfläche eingelassenes Mitnahmeprofil, insbesondere einen Innensechskant (18) oder einen Innenvierkant zum Ansetzen eines Einschraubwerkzeuges aufweist.

16. Hüftgelenkschale nach einem der Ansprüche 12 bis 15, wobei die Hüftgelenkschale (1) und/oder die Verschlussschraube (17) aus einem Polymer, insbesondere aus einem mit Titan beschichteten Polymer gefertigt sind.

17. Hüftgelenkschale nach einem der Ansprüche 12 bis 16 umfassend wenigstens eine Knochenschraube (19) mit einem zum sphärischen Abschnitt (9) der Hüftgelenkschraube korrespondierenden sphärischen Schraubenkopfabschnitt (21), wobei die Schraubenöffnung (4) sowohl bei eingeschraubter Verschlussschraube (17), als auch bei eingesetzter Knochenschraube (19) fluiddicht verschliessbar ist.

18. Hüftgelenkschale nach einem der Ansprüche 1 bis 17, umfassend wenigstens eine Knochenschraube (19), wobei die Schraubenöffnung (4) und der Schraubenkopf (20) der Knochenschraube derart ausgebildet sind, dass die Knochenschraube bezogen auf die Längsmittelachse (12) der Schraubenöffnung (4) in verschiedenen Winkelstellungen einschraubbar ist, ohne dass der Schraubenkopf (20) in die Innenseite (2) ragt.

19. Hüftgelenkschale nach einem der Ansprüche 1 bis 18, wobei zwischen dem äquatorialen Bereich (6) und dem Polbereich (7) in Umfangsrichtung mehrere Schraubenöffnungen (4, 4'), vorzugsweise drei bis fünf Schraubenöffnungen angeordnet sind.

## Claims

1. Hip joint cup (1) with an inner face (2) and an outer face (3) and with at least one screw opening (4) which is intended for the insertion of a bone screw (19) and which has at least one spherical portion, tapering in cross section towards the outer face and serving as abutment for the bone screw, and at least one inner thread (8) for closing the screw opening with a closure screw (17), **characterized in that** the inner thread (8) is arranged in the spherical portion (9).

2. Hip joint cup according to Claim 1, wherein the inner thread (8) is a spherical or a conical or a cylindrical thread.

3. Hip joint cup according to Claim 1 or 2, wherein the inner thread (8) is designed in such a way that, with respect to the cross section, spherical subsidiary portions (26) remain between individual thread turns (11).

4. Hip joint cup according to one of Claims 1 to 3, wherein the spherical portion (9) is adjoined, towards the outer face (3), by a cylindrical portion (10), of which the length (1) preferably measures 0.1 to 0.4 mm.

5. Hip joint cup according to Claim 4, wherein the inner thread (8) does not extend as far as the cylindrical portion (10).

6. Hip joint cup according to one of Claims 1 to 5, wherein the spherical portion (9) extends as far as the inner face (2).

7. Hip joint cup according to one of Claims 1 to 6, wherein the inner thread is arranged in such a way that, with respect to the screwing-in direction, the start (15) of the inner thread is arranged at a distance from the greatest diameter (D) of the spherical portion (9).

8. Hip joint cup according to one of Claims 1 to 7, wherein the inner thread (8) is a single-start thread with at least one complete thread circumference.

9. Hip joint cup according to one of Claims 1 to 7, wherein the inner thread (8) is a multi-start thread, in particular a two-start thread or a three-start thread.

10. Hip joint cup according to one of Claims 1 to 9, wherein the inner thread (8) is an angular thread.

11. Hip joint cup according to Claim 10, wherein the angle bisector (25) of the flank angle (α) with respect to the longitudinal centre axis (12) of the screw opening (4) is inclined from the inlet side towards the outlet side.

12. Hip joint cup according to one of Claims 1 to 11, comprising at least one closure screw (17) for closing the at least one screw opening (4), wherein the closure screw has an outer thread (16) which can be screwed into the inner thread (8), and at least one spherical outer portion (13) corresponding to the spherical portion (9) of the screw opening (4).

13. Hip joint cup according to Claim 12, wherein the outer thread (16) of the closure screw (17) is arranged in the spherical outer portion (13).

14. Hip joint cup according to Claim 12 or 13, wherein the closure screw (17) is designed as a disc whose height (H) is smaller than the entire axial length (L) of the screw opening (4).

15. Hip joint cup according to one of Claims 12 to 14, wherein the closure screw (17) has a driving profile let into a surface, in particular a hexagon socket (18) or a square socket for the attachment of a screwing-in tool.

16. Hip joint cup according to one of Claims 12 to 15, wherein the hip joint cup (1) and/or the closure screw (17) are/is made from a polymer, in particular from a titanium-coated polymer.

17. Hip joint cup according to one of Claims 12 to 16, comprising at least one bone screw (19) with a spherical screw head portion (21) corresponding to the spherical portion (9) of the hip joint screw, wherein the screw opening (4) is closable in a fluid-tight manner both when the closure screw (17) is screwed in and also when the bone screw (19) is inserted.

18. Hip joint cup according to one of Claims 1 to 17, comprising at least one bone screw (19), wherein the screw opening (4) and the screw head (20) of the bone screw are designed in such a way that the bone screw can be screwed in at different angular settings with respect to the longitudinal centre axis (12) of the screw opening (4), without the screw head (20) protruding into the interior (2).

19. Hip joint cup according to one of Claims 1 to 18, wherein a plurality of screw openings (4, 4'), preferably three to five screw openings, are arranged circumferentially between the equatorial region (6) and the pole region (7).

## Revendications

1. Cupule d'articulation de la hanche (1) comprenant un côté intérieur (2) et un côté extérieur (3) et au moins une ouverture de vis (4) prévue pour l'insertion d'une vis à os (19), qui présente au moins une portion sphérique rétrécissant la section transversale vers le côté extérieur en tant que butée pour la vis à os et au moins un filetage intérieur (8) pour fermer l'ouverture de vis avec une vis de fermeture (17), **caractérisée en ce que** le filetage intérieur (8) est disposé dans la portion sphérique (9).

2. Cupule d'articulation de la hanche selon la revendication 1, dans laquelle le filetage intérieur (8) est un filetage sphérique ou conique ou cylindrique.

3. Cupule d'articulation de la hanche selon la revendication 1 ou 2, dans laquelle le filetage intérieur (8) est réalisé de telle sorte que par rapport à la section transversale, entre deux filetages individuels (11) subsistent des portions partielles sphériques (26).

4. Cupule d'articulation de la hanche selon l'une quelconque des revendications 1 à 3, dans laquelle une portion cylindrique (10) se raccorde à la portion sphérique (9) vers le côté extérieur (3), sa longueur (1) valant de préférence 0,1 mm à 0,4 mm.

5. Cupule d'articulation de la hanche selon la revendication 4, dans laquelle le filetage intérieur (8) ne s'étend pas jusqu'à la portion cylindrique (10).

6. Cupule d'articulation de la hanche selon l'une quelconque des revendications 1 à 5, dans laquelle la portion sphérique (9) s'étend jusqu'au côté intérieur (2).

7. Cupule d'articulation de la hanche selon l'une quelconque des revendications 1 à 6, dans laquelle le filetage intérieur est disposé de telle sorte que, par rapport à la direction de vissage, le début (15) du filetage intérieur soit disposé à distance du plus grand diamètre (D) de la portion sphérique (9).

8. Cupule d'articulation de la hanche selon l'une quelconque des revendications 1 à 7, dans laquelle le filetage intérieur (8) est un filetage à un pas avec au moins un pas de filetage périphérique complet.

9. Cupule d'articulation de la hanche selon l'une quelconque des revendications 1 à 7, dans laquelle le filetage intérieur (8) est un filetage à plusieurs pas, en particulier un filetage à deux pas ou un filetage à trois pas.

10. Cupule d'articulation de la hanche selon l'une quelconque des revendications 1 à 9, dans laquelle le filetage intérieur (8) est un filetage triangulaire.

11. Cupule d'articulation de la hanche selon la revendication 10, dans laquelle la médiatrice (25) de l'angle de flanc (a) par rapport à l'axe médian longitudinal (12) de l'ouverture de vis (4) est inclinée depuis le côté d'entrée vers le côté de sortie.

12. Cupule d'articulation de la hanche selon l'une quelconque des revendications 1 à 11, comprenant au moins une vis de fermeture (17) pour fermer l'au moins une ouverture de vis (4), la vis de fermeture présentant un filetage extérieur (16) qui peut être vissé dans le filetage intérieur (8), ainsi qu'au moins une portion extérieure sphérique (13) correspondant à la portion sphérique (9) de l'ouverture de vis (4).

13. Cupule d'articulation de la hanche selon la revendication 12, dans laquelle le filetage extérieur (16) de la vis de fermeture (17) est disposé dans la portion extérieure sphérique (13).

14. Cupule d'articulation de la hanche selon la revendication 12 ou 13, dans laquelle la vis de fermeture (17) est réalisée sous forme de rondelle dont la hauteur (H) est inférieure à la longueur axiale totale (L) de l'ouverture de vis (4).

15. Cupule d'articulation de la hanche selon l'une quelconque des revendications 12 à 14, dans laquelle la vis de fermeture (17) présente un profil d'entraînement incorporé dans une surface, en particulier un profil à six pans creux (18) ou un profil à quatre pans creux pour l'application d'un outil de vissage.

16. Cupule d'articulation de la hanche selon l'une quelconque des revendications 12 à 15, la cupule d'articulation de la hanche (1) et/ou la vis de fermeture (17) étant fabriquées à partir d'un polymère, en particulier d'un polymère revêtu de titane.

17. Cupule d'articulation de la hanche selon l'une quelconque des revendications 12 à 16, comprenant au moins une vis à os (19) avec une portion de tête de vis sphérique (21) correspondant à la portion sphérique (9) de la vis d'articulation de la hanche, l'ouverture de vis (4) pouvant être fermée de manière étanche aux fluides à la fois lorsque la vis de fermeture (17) est vissée et lorsque la vis à os (19) est insérée.

18. Cupule d'articulation de la hanche selon l'une quelconque des revendications 1 à 17, comprenant au moins une vis à os (19), l'ouverture de vis (4) et la tête de vis (20) de la vis à os étant réalisées de telle sorte que la vis à os, par rapport à l'axe médian longitudinal (12) de l'ouverture de vis (4), puisse être vissée dans différentes positions angulaires sans que la tête de vis (20) ne pénètre dans le côté intérieur (2).

19. Cupule d'articulation de la hanche selon l'une quelconque des revendications 1 à 18, dans laquelle, entre la région équatoriale (6) et la région polaire (7), dans la direction périphérique, sont disposées plusieurs ouvertures de vis (4, 4'), de préférence trois à cinq ouvertures de vis.
